# EUROPEAN PATENT APPLICATION

(11) **EP 2 366 417 A2**
(43) Date of publication of application: **21.09.2011**
(21) Application number: 11157648.4
(22) Date of filing: 10.03.2011
(51) Int. Cl.: A61M 5/32, B02C 19/00

(54) **Device for cutting needles for sanitary use**

(30) Priority: 17.03.2010 IT PS20100008
(71) Applicant: Frongia, Stefano, 61029 Urbino (PU) (IT)
(72) Inventor: Frongia, Stefano, 61029 Urbino (PU) (IT)
(74) Representative: Gustorf, Gerhard

(57) **Abstract**

Device for cutting needles for sanitary use comprising an automatic guillotine (9, 10) housed concealable (2) immediately at the surface suitable to cut - at the root and piercing e/o incising instrument for sanitary use of any type inserted without contaminating contacts in a sensor-equipped (16) shaped hole (11) of the housing (2) and collected in a safety container (3).

## Description

### Prior art

Statics reveal that about 75% of the accidents at work that occur in the sanitary field, among health operators, are linked to accidental pricking caused by needles which, if occurring after respective use, may lead to consequences particularly harmful due to the injured person contracting virus.

Currently, measures against such injuries, apart from the practical (and also a source of accidents) sheathing of the needle after respective use by means of needle guards, are represented by devices that melt the needle, but such machines require developing extremely high temperatures, with ensuing production limits due to the small number of needles treatable within a period of time, as well as drawbacks due to the heat and odour transmitted to the external environment.

In order to overcome such drawbacks the same current applicant already conceived a solution concept that provides for milling the needle, according to the industrial invention patent n° 1.332.605 granted on 7th March 2006 based on application n° PS2002A000003 filed on 25^{th} January 2002, but such solution turned out to be noisy and not suitable to be used in hospitals, retirement homes and hospitalization places in general.

Also alternative solutions exist, which provide the cutting of the needle, which are shown in the US patent No. US5761975 in the name of Casey E. Waluda and in the French Patent Application publication No. 2 756 492 and international publication No. W09824497 in the name of Jacques Granier, but such solutions are not multipurpose for every kind of needle and piercing and/or incising instrument for sanitary use and furthermore they are not transportable.

### Objects of the invention

Therefore, the main object of the present invention, within the abovementioned context, is that of providing a solution concept suitable to attain the cutting of the needles for sanitary use overcoming the limits and drawbacks of the solutions according to the prior art, i.e. such to attain high productivity without contraindications in terms of disturbance of any kind whatsoever and usable in any environment.

Another object of the present invention is that of attaining the preceding object simultaneously meeting all the most demanding standards in terms of safety, storage and disposal of special hospital waste.

Another object of the present invention is that of attaining the preceding objects simultaneously achieving a multipurpose instrument for any and every kind of needle and piercing and/or incising instrument for sanitary use.

A further object of the present invention is that of obtaining the preceding objects through a device that is simple and efficient, operating safely and being relatively inexpensive considering the results practically obtained therewith.

### Summary of the solution concept

These and other objects are all attained by means of the device for cutting needles for sanitary use according to the present invention, comprising an automatic guillotine (9, 10) housed concealable (2) immediately at the surface suitable to cut - at the root needle and piercing and/or incising instrument for sanitary use of any type inserted without contaminating contacts in a sensor-equipped (16) shaped hole (11) of the housing (2) and collected in a safety container (3).

### Description of the attached drawings

Further characteristics and advantages of the device for cutting needles for sanitary use according to the present invention shall be clearer from the detailed description that follows of a preferred but not exclusive embodiment thereof, strictly represented for exemplifying and non-limiting purposes in the three attached drawings, wherein:
Figure 1 shows an exploded perspective view of the operative unit of the device for cutting needles for sanitary use according to the present invention.

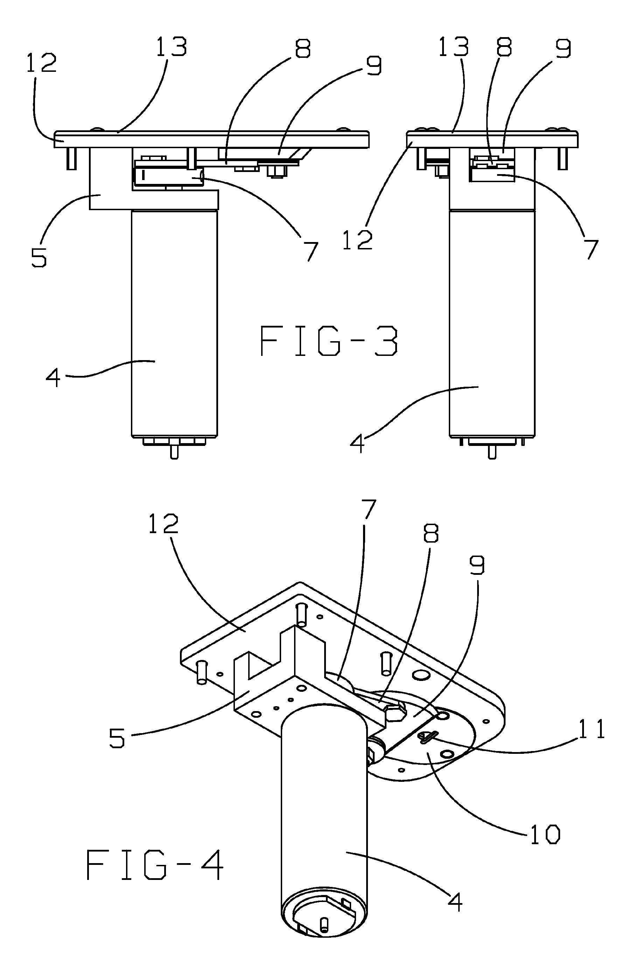

Figures 2 to 4 respectively show an elevational perspective view, a side profile view, a rear profile view and a bottom perspective view of the assembly of the operative unit of the device for cutting needles for sanitary use according to the present invention.
Figure 5 shows the operative unit of the device for cutting needles for sanitary use according to the present invention as inserted in the respective box-shaped container and connected to the safety collector of the cut needles.

### Static description of the embodiment

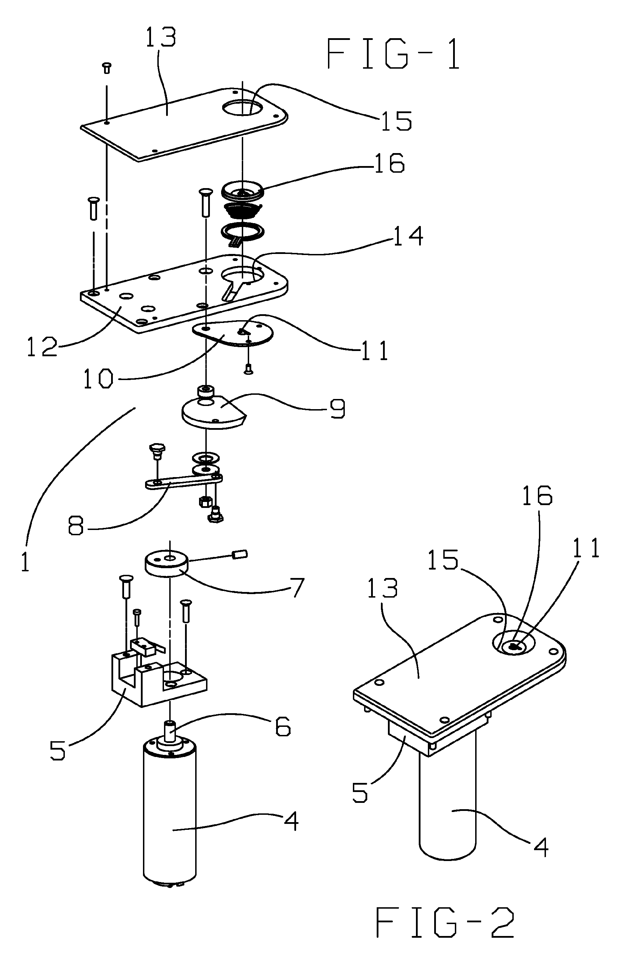

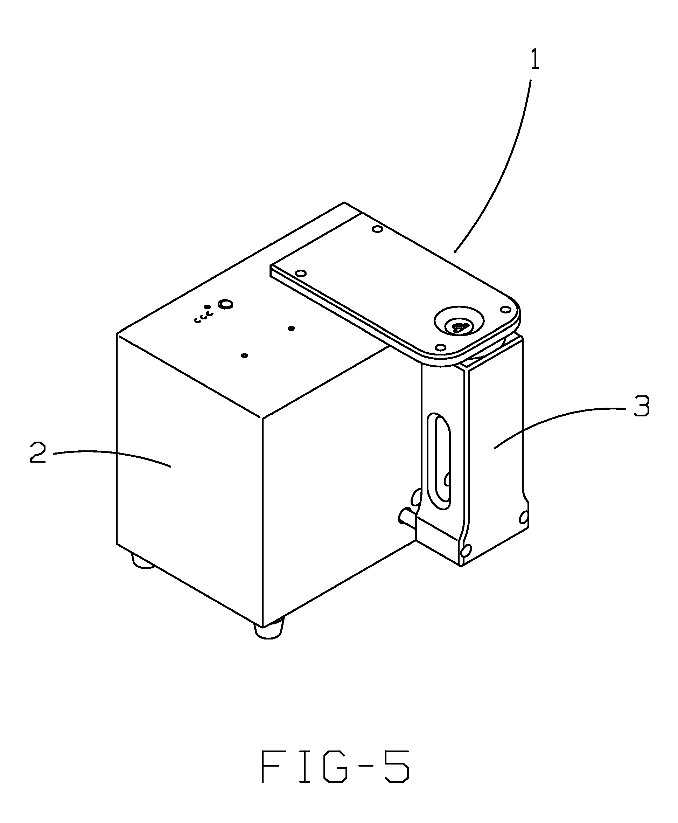

Referring to such figures, and in particular Figures 1 and 5, indicated in its entirety with 1 is the operative unit of the device for cutting needles for sanitary use according to the present invention, and indicated with 2 is a box-shaped container where it is inserted, which also accommodates a rechargeable electric battery, not illustrated, and it is associated to a safety collector 3 of the cut needles, not illustrated, collected in disposable removable container inserted therein, also not illustrated.

The operative unit 1 is made up of an electric motor 4, fixed beneath a casing 5, with a vertical motor axis 6, associated to an eccentric 7 at the upper part.

The eccentric 7 actuates a connecting rod 8, connected at the other end to a cutting head 9, fulcrumed beneath a lower plate 12 by interposing a fixed counterblade 10, having a hole 11 suitably dimensioned and shaped for introducing needles for sanitary use of any diameter and disposable bistoury blades of every shape.

The lower plate 12 is sandwich-like assembled against an upper plate 13 and interposed therebetween is an electric pressure sensor, entirely indicated including its components with 16, accommodated in circular slots 14 and 15 coaxially obtained in the plates 12 and 13 and in the shaped hole 11 of the fixed counterblade 10, for the purposes outlined hereinafter.

### Dynamic description of the embodiment

Thus, having completed the static description of a preferred embodiment of the device for cutting needles for sanitary use according to the present invention, following is the dynamic description thereof, i.e. the respective operation:

The box-shaped container 2, comprising the operative unit 1 of the device for cutting needles for sanitary use according to the present invention, may be located in any sanitary station using needles and/or disposable bistoury, in particular it may be located in mobile sanitary stations, such as trolleys used in hospital wards for administering therapies, due to the small dimensions thereof and the fact that it is power-supplied by a rechargeable battery, which allows several uses definitely meeting the needs of an ordinary therapeutic shift in a ward.

In such context, once a needle, of any type, has been used and it is removed from the body of the patient, or a disposable bistoury has completed its function, it is immediately inserted by the health operator into the hole 11 of the counterblade 10 without any intermediate passage;

In such manner, the root of the inserted needle and the respective body for fixing the health instrument into which it has access, whichever it is, or the distal portion of the handle of a disposable bistoury interferes with the electric pressure sensor 16, surrounding the shaped hole 11, which, thus pressed, commands the actuation of the motor 4, which, by rotating around its axis 6, actuates the eccentric 7 and, through the connecting rod 8, it imparts an excursion to the cutting head 9, which cuts off the needle projecting at the lower part of the hole 11 beneath the fixed counterblade 10.

The cut needle falls into the safety collector 3 which, when full, for example after a preset number of operative cycles of the unit 1, may be opened by safely removing the disposable removable container inserted therein to be taken to the collection points of the special hospital wastes.

The positioning of the cutting head 9 at immediate surface level, under the plates 13 and 14 which close the device on the upper side, without any insertion channel but under a plane open to all orientations, permits the use of the device for any kind and type of needle and bistoury, whatever be the shape of the relevant handling portion.

### Alternative embodiment

It is obvious that further alternative embodiments still falling within the concept solution under the embodiment illustrated above and claimed hereinafter, the device for cutting needles for sanitary use according to the present invention may be contained using equivalent technical and mechanical materials, i.e. provided with further supplementary solutions, same case applying to all configurations of the respective components which may vary to suit the purpose.

### Advantages of the invention

As observable from the detailed description above regarding a preferred embodiment, the device for cutting needles for sanitary use according to the present invention offers advantages corresponding to the attainment of the preset objects and even others: as a matter of fact, it integrates a functional, modular, polyvalent and economical automatic device suitable to eliminate any type of needle and disposable bistoury blade from the respective support in any kind of sanitary device immediately after use, at any place of use, thus eliminating - at the root - any possibility of contact between the operators and third parties and respective risks of infection.

Specific advantage with reference to the device according to the know art, which substantially allow the cutting of needles applied to cylindrical syringes only, results from the positioning of the cutting head at immediate surface level, which allows the cutting of needles with support of any kind, for example hypodermic butterfly needles, fistula needles, introducer kits, catheter needles, introducer needles, blood vacuum collection needles, which never can reach to get into till the cutting means of the devices according to the known art.

Furthermore the device according to the present invention can be placed on mobile sanitary stations, such as trolleys used in hospital wards for administering therapies, due to the fact that it is power-supplied by a rechargeable battery; in so far a fixed device for cutting needles for sanitary use is substantially useless, being necessary to take needles until the fixed device, with increasing during the transfer of those risk which the device is aimed to avoid at all.

### Scope of protection

Thus, having described the device for cutting needles for sanitary use according to the present invention with reference to a preferred embodiment thereof obviously requires protecting all possible embodiments with variants of implementations known to those skilled in the art, which shall not modify the invention without departing from the objects provided for by the same; this implies, both in the description above and in the claims that follow, protecting all embodiments and variants that fall within the concept solution, for the purpose and objects of the invention itself.

### KEY TO REFERENCE NUMBERS

1) operative unit of the cutting device in its entirety
2) box-shaped container
3) safety needles collector
4) electric motor
5) casing
6) motor axis
7) eccentric
8) connecting rod
9) cutting head
10) fixed counterblade
11) shaped hole for insertion of needles and disposable bistoury blades
12) lower plate
13) upper plate
14) circular slot in lower plate
15) circular slot in upper plate
16) electric pressure sensor in its entirety

## Claims

1. Device for cutting needles for sanitary use, **characterised in that** it comprises an automatic guillotine (9, 10) housed concealable (2) immediately at the surface suitable to cut - at the root and piercing e/o incising instrument for sanitary use of any type inserted without contaminating contacts in a sensor-equipped (16) shaped hole (11) of the housing (2) and collected in a safety container (3).

2. Device for cutting needles for sanitary use according to claim one, **characterised in that** it comprises an operative unit (1) made up of:
- a motorisation (4) associated to means (7) for transforming the rotational motion into a linear motion;
- cutting means (9) placed immediately under the housing surface connected through means for transferring the linear motion (8) to said means (7) for transforming the rotational motion into a linear motion;
- abutment means (10) interacting with said cutting means (9) provided with a shaped hole (11) suitable for the introduction of needles for sanitary use and disposable bistoury blades;
- a sensor (16) for detecting the presence of said needles (16) associated to said shaped hole (11).

3. Device for cutting needles for sanitary use according to claim one or two, **characterised in that** it comprises operative unit (1) made up of:
- an electric motor (4) with vertical axis (6) associated at the top part to an eccentric (7);
- a cutting head (9) placed immediately under the housing surface connected through a connecting rod (8) to said eccentric (7);
- a fixed counterblade (10) interacting with said cutting head (9) having a shaped hole (11) suitably dimensioned for introducing needles for sanitary use and disposable bistoury blades;
- an electric pressure sensor (16) interposed between plates (12, 13) assembled sandwich-like at the height of circular slots 14 and 15 coaxially obtained in said plates (12, 13) at the height of the shaped hole (11) of the fixed counterblade (10), said two plates (12, 13) also representing means for supporting said fixed counterblade (10), of said cutting head (9), of said kinematisms (7, 8) for actuating said cutting head, as well as of said electric motor (4).

4. Device for cutting needles for sanitary use according to any one of the preceding claims, **characterized in that** said operative unit (1) is inserted into a box-shaped container (2) also a accommodating a rechargeable electric accumulator and associated to a safety collector (3) for the cut needles collected in disposable removable container inserted therein.

5. Device for cutting needles for sanitary use according to the preceding claims, **characterized in that** said sensor (16) for introducing needles is of any type suitable for the purpose.

6. Device for cutting needles for sanitary use according to the preceding claims, **characterised in that** it comprises a device for detecting the filling of the cut needles container of any type suitable for the purpose.

7. Device for cutting needles for sanitary use according to the preceding claims and substantially as described and illustrated in the attached drawings as well as for the specified purposes.
